(19) Europäisches Patentamt / European Patent Office / Office européen des brevets

(11) **EP 3 827 805 A1**

(12) **EUROPEAN PATENT APPLICATION**

(43) Date of publication:
02.06.2021 Bulletin 2021/22

(51) Int Cl.:
*A61K 8/02* *(2006.01)*      *A61K 8/73* *(2006.01)*
*A61Q 19/00* *(2006.01)*

(21) Application number: 19212200.0

(22) Date of filing: 28.11.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(71) Applicant: BASF SE
67056 Ludwigshafen am Rhein (DE)

(72) Inventors:
• MUELLER, Jan Ole
67056 Ludwigshafen (DE)

• OSCHMANN, Bernd Dieter
67056 Ludwigshafen (DE)
• WITTELER, Helmut
67056 Ludwigshafen (DE)
• KUENNE, Holger
67056 Ludwigshafen (DE)

(74) Representative: BASF IP Association
BASF SE
G-FLP-C006
67056 Ludwigshafen (DE)

(54) **BIODEGRADABLE BEADS BASED ON CELLULOSE**

(57) The present invention relates to biodegradable spherical beads comprising amorphous cellulose as carrier and a high load of cosmetic ingredients, preferably oils.

Depending on the properties and composition of the beads, they can be used for an easy and convenient application of cosmetic emollients or as peeling agent and cleaning agent in the skincare field.

## Description

The present invention relates to biodegradable spherical beads comprising amorphous cellulose as carrier and a high load of cosmetic ingredients, preferably oils.

Various states have already introduced initiatives to ban microplastics especially in cosmetic products. As of January 2018 the use of microplastic particles and plastic microbeads in personal care products has been prohibited in the United States and by the British government, the EU is following with similar bans.

Microplastics are defined as synthetic polymers below 5 mm in size which are not biodegradable. Microplastic particles are extremely harmful to marine life not only because of their morphological properties but also due to the adsorption of environmental toxins on their large surface area by physico-chemical adhesion of the plastic material.

Among other areas microplastics have been used in cosmetic and personal care compositions as scrubbing and peeling agents for removing the aging keratin on the skin, sebum or residues of face make up and for improving the blood flow in the skin and activating its metabolism for maintaining a healthy skin.

Being colored these particles provided a splendid look to cosmetic compositions and tempt users to feel good when using exactly this composition. Using colored microplastics it was possible to cover unwanted grey, brown or impure looking adjuvants in cosmetic compositions.

Beyond doubt these advantages do not outweigh the negative implications microplastics have on our environment. These microbeads added to cosmetics and personal care products have meanwhile been replaced by natural material, such as for example ground nut shells, apricot kernels, minerals, waxes or biodegradable polymers.

It was one object of the invention to provide visible particles or beads for cosmetic and personal care products which do not have a negative impact on our environment and could substitute hazardous microplastic beads. Preferably these particles should be transparent for cosmetic use.

A comprehensive overview on cellulose beads is been given in a review article by Martin Gericke et al. "Functional Cellulose Beads: Preparation, Characterization, and Applications" published 2013 (013 American Chemical Society Publications - Chem. Rev. 2013, 113, p. 4812-4836). The described beads are used in the field of chromatography, ion exchange and water treatment, protein immobilization and pharmaceutical drug delivery.

Their cosmetic use has not been investigated in detail. In this area mainly cellulose partices made by wet granulation are known.

Exfoliating beads with a particle size in the range of 1 $\mu$m to 900 $\mu$m comprising different cosmetic ingredients have been prepared by wet granulation according to European patent application EP 2 907 498 A1. Sufficient stability of these beads has only be achieved with an ingredient concentration of up to 10 weight % based on the cellulose particle.

In addition the bead used in the cosmetic field may carry cosmetic ingredients and enable the user to easily apply these ingredients to the skin.

The US application US 2018/0280424 discloses solid beads comprising prebiotics as active solid ingredients and fillers such as cellulose. They are also manufactured by wet granulation. The actives could be applied through the skin by rubbing the beads and subsequent absorption of the active through the skin.

The European patent EP2190882 discloses a process for the production of spherical, non-fibrillar cellulose particles with a particle size in the range of 1 mm to 400 mm from a solution of non-derivatised cellulose in a blend of an organic substance and water characterised in that the solution is cooled down to below its solidification temperature free flowing, the solvent is washed out, the solidified cellulose solution is disintegrated, the solvent is washed out and the disintegrated and washed out particles are dried. The resulting particles are described as remotely spherical with a width to length ratio of 1 - 2.5, but far from round particles with smooth surface. The mechanical milling steps result in an undefined rough surface which are claimed to be beneficial for the desired application. The crystallinity of the resulting particles is given as 15 - 35 % which suggests that the described particles are not translucent.

Taro Omura et al. - published as "Encapsulation of Either Hydrophilic or Hydrophobic Substances in Spongy Cellulose Particles"; American Chemical Society - ACS Appl. Mater. Interfaces 2017, 9, p. 944-949 - have manufactured cellulose particles comprising a fluorescent substance in a spongy structure. Storing these beads at room temperature they collapse because the solvent inside them evaporates. 1-butanol is used as precipitation medium, so that these beads can not be used in pharmaceutical and cosmetic compositions. The beads are dried with supercritical carbondioxide to develop the spongy structure which enables the absorption of a dye in toluol or water.

The US application US 2004/0131690 discloses beads comprising a carrier liquid and flocculated microcrystalline cellulose which are suitable for use in cosmetic applications. By rubbing they break down completely to release the entrapped material and leave no residue as on the skin. The flocculated cellulose beads require the presence of a flocculation medium such as alginate or carrageen and a salt. Because of stability reasons it is recommended to store these beads in a dispersion medium such as deionized water. A storage of the pure beads at room temperature without the aqueous environment is not described as feasible and results in shrinkage or stability problems. The described particles contain cellulose microcrystals and can consequently not be translucent.

James Coombs O'Brien et al. ("Continuous Production of Cellulose Microbeads via Membrane Emulsification", American Chemical Society, ACS Sustainable Chem. Eng. 2017, 5, pages 5931 to 5939) have investigated cellulose beads prepared by emulsification techniques. A solution of cellulose in DMSO and 1-ethyl-3-methylimidazolium acetate has been dispersed in sunflower oil containing 2 weight % emulsifier for providing the lipophilic phase of the emulsion, which has then been dropped into ethanol to achieve cellulose beads. The sunflower oil is a vehicle only allowing the simple setting of the beads, it is not absorbed by the beads during or after precipitation. The beads are washed with ethanol. Due to the dissolution medium being an imidazolium salt in DMSO these beads were not suitable for cosmetic or personal care products. Beads resulting from the emulsification technique reach a diameter of about 100 $\mu$m. In order to achieve beads of mm-size the cellulose beads were crosslinked with glyoxal. Crosslinking does not only result in larger beads but also in hardening the surface and a remarkable increase of the pressure resistance of the beads.

The main object of the invention was the delivery of emollients to the skin in form of easily processible solid beads with a high loading and increased storage stability. On the one hand these beads should be easy to handle and stable enough for the incorporation in personal care compositions during the shelflife of cosmetic compositions. On the other hand they should break down and release the incorporated emollient upon distribution or application of the cosmetic composition on the skin. It should be possible to store the beads without a dispersion medium.

**Detailed description of the invention**

These objects were solved by providing spherical beads with a diameter of D(4,3) of 1 - 5000 $\mu$m comprising 1 to 80 % by weight of amorphous cellulose,

at least 20 % by weight of a cosmetic ingredient with a molecular weight > 50 g/mol;
and less than 10 % by weight of water based on the weight of the beads.

The beads of the invention have a specific physical stability as they are stable at room temperature outside a dispersion medium but collapse when they are subjected between two parallel plates administrating a force of at least 0.005 N. This stability enables storage of the beads at room temperature without the use of dispersion media, an easy handling of the beads for further processing as well as transport and possibilities to use them in in-situ-preparations of the customer/end-user.

Nevertheless, the beads rupture by applying a force with the user's hand or fingers to spread the incorporated cosmetic ingredient on the skin, mucosa or hair. After release of the entrapped ingredients the latter could easily and evenly be distributed on the skin and result in a pleasant and soft skin feeling.

The beads could as well be used as a peeling agent because their abrasive properties can be adjusted by the amount of cellulose and the type and amount of incorporated agents. Soft beads comprise a lower amount of cellulose and a higher amount of oils or emollients while harder beads with a higher concentration of cellulose and lower loading capacity have better abrasive properties.

Depending on the use of the beads, for example the distribution of emollients for hydration of the skin and a smooth skin feeling or the use as peeling agent with abrasive properties for cleaning the skin the composition of the beads can be adjusted.

Measurement of the collapsing force:

**Description of texture analyzing method:**

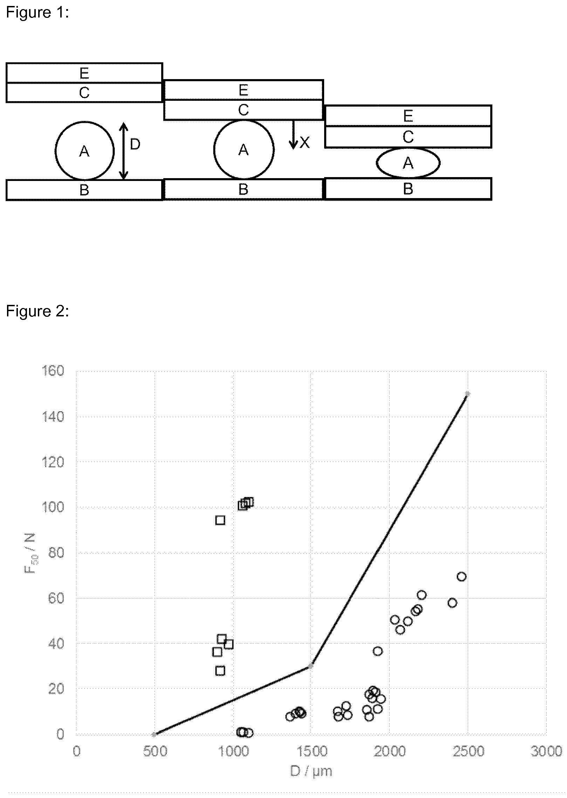

The diameter (D) of a bead (A) is determined by use of an optical microscope (see "Determination of particle size distribution by light microscopy") . The deformation curve of the bead is measured with a Texture Analyzer TA.XT by WINOPAL (Forschungsbedarf GmbH, Elze, Germany). For this measurement (see figure 1), a single bead (A) of a defined diameter (D) is placed on a flat surface (B) under the centre of a flat stamp (C) of 32 mm diameter, which is parallel to surface (B) and it is equipped with a pressure sensor (E). Stamp (C) is lowered with a speed of 2 mm/s until the sensor (E) registers a force of 0.05 N (figure 1). After that, the speed of the stamp is reduced to 1 mm/s and the recording of the compression force (F) as measured by (E) starts. At the same time, the displacement (X) of (C) is recorded. For data evaluation, (F) is plotted versus (X). The force ($F_{50}$) that is required to compress a bead to 50% of its initial diameter (X = 0.5 x D) is retrieved from the recorded data.

Preferably the beads according to the invention are characterized in that their deformation between two parallel plates obeys to the rules

$F_{50}$ < 0.03 x D -14.95 for beads of D(4,3) of 500 to 1500 $\mu$m and
$F_{50}$ < 0.12 x D -149.95 for beads of D(4,3) of 1500 to 3000 $\mu$m

with $F_{50}$ being the force measured in N necessary to compress the bead to 50% of its original diameter D measured in $\mu$m and wherein $F_{50}$ is larger than 0.05 N.

More preferably the beads of the invention are characterized in that they have a diameter of 500 to 3000 $\mu$m and their deformation between two parallel plates obeys to the rules

$F_{50} < 0.03 \times D -14.95$ for beads of D(4,3) of 500 to 1500 $\mu$m and
$F_{50} < 0.12 \times D -149.95$ for beads of D(4,3) of 1500 to 3000 $\mu$m
with $F_{50}$ being the force measured in N necessary to compress the bead to 50% of its original diameter D measured in $\mu$m and wherein $F_{50}$ is larger than 0.1 N.

Figure 2 shows the 50% compression of beads. Plot of the force necessary to compress a bead by 50% of its initial diameter ($F_{50}$) plotted versus the initial diameter (D) of the bead. Inventive examples (circles), comparative examples (squares: comparative material "Vivapor CS 800 S" and example 2.2). Examples of the collapsing force are listed in table 4.

**Beads Size**

Measurement of the bead's diameter can be conducted by laser diffraction and by light microscopy.

Determination of particle size distribution by laser diffraction:

Particle Size Distribution (PSD) was determined by laser diffraction using a Malvern Mastersizer 2000 according to European norm ISO 13320:2009 EN. The data were treated according to the Mie-Theory by software using a "universal model" provided by Malvern Instruments. Important parameters are in particular the following values: D(v 0,5), D(v 0,9), D(v 0,1), D(3,2) and D(4,3), where D(v 0,5), D(v 0,9), D(v 0,1), D(3,2) and D(4,3) are as defined herein. In the context of particle size, the D(0,9) or D(v 0,9) value of particle size indicates that 90 vol.-% of the particles have a hydrodynamic diameter smaller than this value. In the context of particle size, the volume median particle diameter D(0,5) or D(v 0,5) value, respectively, means that 50 vol.-% of the particles have a diameter which is above the value cited and 50 vol.-% of the particles have a diameter which is below the value cited. In the context of particle size, the D(0,1) or D(v 0,1) value indicates that 10 volume % of the particles have a hydrodynamic diameter below the value cited. The D(3,2) describes the surface-weighted average mean of all particles, while the D(4,3) describes the volume-weighted average mean of all particles.

Determination of particle size distribution by light microscopy:

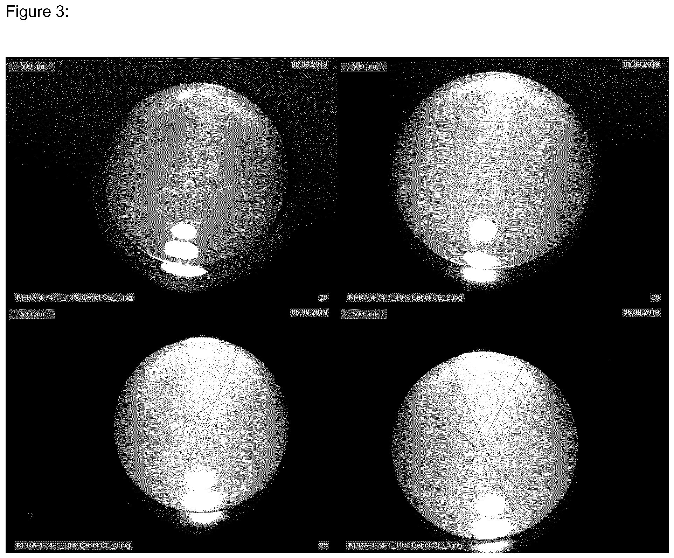

Particle Size Distribution (PSD) was determined by optical light microscopy (Leica Z16 APO zoom-system + Leica DFC420 digital color camera and Leica KL 1500 LCD light source) and manual measurement (double determination) of the diameter of 4 - 10 representative particles using the software Leica Application Suite (LAS) Core (see figure 3: beads size determination by light microscopy (beads of example 3.16)).

The volume based average particle size D(4,3) was calculated according to formula I:

Formula I

$$D(4,3) = \frac{\sum_1^n Di^4}{\sum_1^n Di^3}$$

The beads of the invention are small solid or semi-solid objects with diameters D(4,3) of 1 to 5000 $\mu$m, preferably 100 to 4000 $\mu$m, more preferably 500 to 3000 $\mu$m, most preferably 1000 to 3000 $\mu$m and especially 2000 to 3000 $\mu$m as determined by light microscopy.

The beads of this invention are "spherical", which means that the ratio between length and width of the beads is between 1 and 2, preferably between 1 and 1.5 and more preferably between 1 and 1.2.

**Amount of water**

While beads of the prior art comprise a certain amount of water due to their manufacturing method the amount of water in the inventive beads is very low. It is less than 10 % by weight, preferable less than 5 % by weight, more preferably less than 3 % and most preferably less than 1 % by weight of water based on the weight of the beads. The water content has an influence of the physical and chemical stability of the beads. The lower the amount of water the better is the loading capacity of the oils is.

Determination of water content in cellulose particles

A sample of the cellulose beads is weighted into a glass ampoule and introduced in a 120°C preheated oven (874 Oven Sample Processor, Methrom). The water vapor is transferred into the previously dry-titrated Coulometer (852 Tritrando, Methrom) by a stream of dry nitrogen. The content of water is determined coulometrically by bipotentiometric indication. The minimum time of the measurement is 300 s. A drifting is determined at the beginning of the measurement (startdrift), the measurement is stopped when the drift is equal to a startdrift plus 15 $\mu$g/min, but measurement time is at least 300 s. The relative standard deviation of the determination is below 1%.

**Porosity**

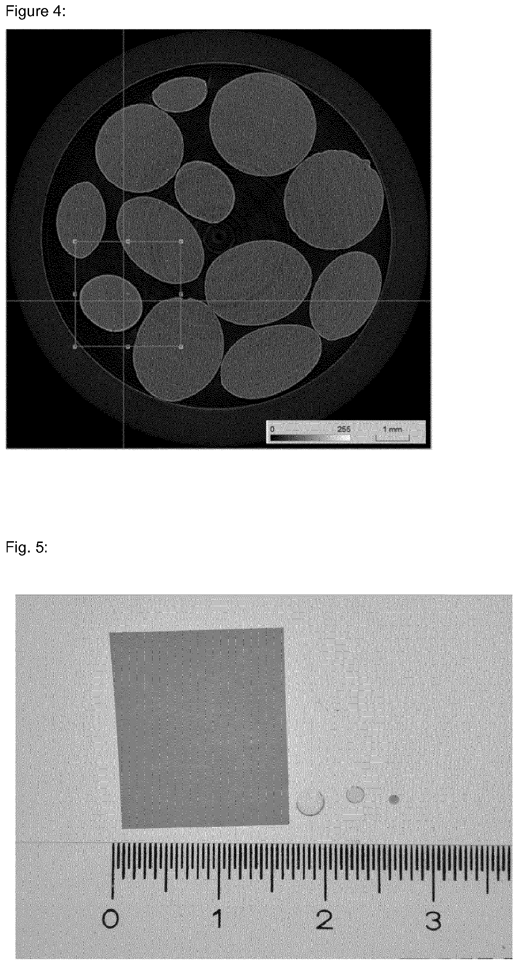

The porosity of the particles has not been investigated further as BET - and CT-measurements have not shown any pores on the surface and inside the beads (see figure 4). The beads are completely homogeneous inside, a porosity cannot be detected. Figure 4 shows A Micro-CT measurement of cellulose beads of example 2.1 of the cross section of particles.

A BET-measurement (Instrument: Gemini VII, Micromeritics, Nitrogen absorption for 24 h at 23°C, relative pressure range $p/p_0$ of 0.05 to 0.3) of example 2.1 has given a BET-surface of 0.1756 $m^2$/g with a BET-constant of 7.68.

It is clearly shown, that no pores of a detectable size range can be discovered. This low value for the BET-surface confirms the observation of the $\mu$-CT measurements, where no pores in $\mu$m size range could be observed. The absence of pores in the range of the visible light wavelength would also explain the translucence of the beads. As the beads do not have a porous structure the loading with the dyes and pigments needs to be considered during the processing of the beads before a final drying step. Consequently, loaded beads contain ingredients which are incorporated in the matrix of the beads.

**Translucence**

Preferably the spherical beads are translucent. "Translucent" means that the beads allow light to pass through partially or diffusely. They have a glassy, transparent look and they are permitting light to pass through but diffusing it so that objects on the other side cannot be clearly visible. The terms "translucent" and "translucence" are used in the same meaning as "transparent" and "transparency".

The translucence is only possible if the complete cellulose of the bead is in an amorphous state.

The beads disclosed in the European patent EP2190882 have a degree of crystallinity of 15 - 35% due to the applied solvent system and can consequently not be translucent.

The beads disclosed in US application US 2004/0131690 comprise flocculated microcrystalline cellulose. They are not translucent because of the presence of cellulose crystallites due to their way of manufacturing. Oil-filled beads with flocculated cellulose are manufactured by (a) preparing an aqueous dispersion comprising colloidal microcrystalline cellulose; (b) preparing an oil phase comprising an oil; (c) combining the aqueous dispersion and the oil phase to form an emulsion; and (d) adding portions of the emulsion to a setting bath comprising water and at least one flocculating agent selected from a salt, an organic solvent, a pH modifier or a cationic material. Step a) comprises the dispersion of colloidal microcrystalline cellulose and this colloidal microcrystalline cellulose is then flocculated by the flocculating agent, following that the structure of the beads is porous, not homogenous inside and not translucent.

Beads as manufactured according to Taro Omura et al. - ACS Appl. Mater. Interfaces 2017, 9, p. 944-949 - collapse and become opaque after evaporation of the solvent 1-butanol.

Determination of transparency/translucence of cellulose particles with a UV-VIS-spectrophotometer:

Transparent in the sense of this invention is a cellulose bead when it appears brighter than a reference material that exhibits a transparency for visible light of 0.6%. The inventive cellulose beads and the reference material are observed and compared in direct light. "Direct light" means that the specimen and the reference material are placed between the eye of the observer and the source of light. "Light" is visible light or monochromatic light from the visible spectrum.

As reference material for transparency, a flat material with limited transparency is used, here a non-colored stationary paper of a thickness of 80 $g/m^2$. The transparency of the reference material is measured with a UV-VIS-spectrophotometer. The reference material is put into a cuvette perpendicular to the light beam of the spectrophotometer.

Measurement of the transparency of the reference material in figure 5:

The transparency of the reference material (stationary paper of 80 $g/m^2$) is 0.4% at 350 nm, 0.5% at 450 nm and 0.6% at 600 nm. Measured with a spectrophotometer is DR 6000 by Hach Lange GmbH, Düsseldorf, Germany.

Figure 5: Photography (viewed in direct light) of - from left to right - reference material (stationary paper 80 $g/m^2$), inventive material 1 (beads of example 3.15) comparison material 2 (beads of example 2.2) and comparison material cellulose pellet (Vivapur CS 800 S by Rettenmeier, Rosenberg). Samples are placed on a light box (Color Control Professional by Just Normlicht, Weilheim, Germany). Photo is taken with a camera SP-590US by Olympus.

**Active Content**

The beads according to the invention are characterized in that they comprise a very high amount of ingredient while prior art beads have much lower loading capacities. The beads of the invention comprise at least 20 % by weight, preferably at least 35 % by weight, more preferably at least 60 % by weight, most preferably at least 70 % by weight, specifically at least 80 % by weight and most specifically at least 95 % by weight of the cosmetic ingredient based on the weight of the beads.

It is surprising that despite of the high load of cosmetic ingredient, preferably emollient, the beads still have sufficient stability without storage in a dispersion medium.

Determination of active content (*AC*) in cellulose particles according to formula II:

Equipment: 3 l beaker with PTFE half-moon-blade stirrer and three diaphragm pump SIMDOS 02 from KNF.

The beaker was charged with 2000 mL of ethanol. With a rate of 3 mL/min, 193.6 g of cellulose solution in TBAAc/MeCN (2.5 wt.% MCC) were dropped from a height of 0.5 cm to the stirred ethanol. The stirrer was stopped after complete addition of the feed. The obtained cellulose beads sink to the bottom of the breaker immediately upon addition to the ethanol. 1h after completion of the feed, the cellulose beads were isolated by filtration. The cellulose beads were suspended in 2000 mL fresh ethanol for 1 h. This washing step was repeated one more time. Three samples of the ethanol-containing beads were dried in a vacuum oven for 2 h at 120°C. The measured solid content (*SC*) was 3.8 %.

69.1 g of isolated beads (beads swollen with solvent = $B_{solv}$) were added to a mixture of 251.3 g of Cetiol® OE and 249.8 g of ethanol in a 1 L glass beaker. After 1 h the cellulose beads were isolated by filtration and added 502.6 g of fresh Cetiol OE. This step was repeated with 507.1 g of Cetiol® OE. The open vial with Cetiol OE and cellulose beads were stored for 2 days in a vacuum cabinet at 50 mbar and ambient temperature. The cellulose beads were isolated by filtration. The weight of the isolated beads was 62.4 g (beads loaded with active = $B_{active}$). These beads were added to 500.2 g fresh Cetiol®. For the calculation for specific example see formula III.

Formula II:

$$\frac{B_{active} - (B_{solv} \; x \; SC\,\%)}{B_{active}} = AC\,\%$$

Formula III:

$$\frac{62.4 - (69.1 \; x \; 3.8\%)}{62.4} = 95.8\%$$

The beads of the present invention are stable under various conditions. At dry storage under air at ambient conditions the beads maintain there spherical form without significant release of cosmetic ingredient. Even upon storage for at least 9 months the particles are stable without disintegration and no changes to the initial appearance. Storage in aqueous environment is also feasible without disintegration of the beads. The particles maintain their spherical form as well as a significant amount of cosmetic ingredient. The leakage of cosmetic ingredient was not quantified.

**Cellulose**

The beads of the current invention are characterized in that they comprise 1 to 80 % by weight, preferably 1 to 50 % by weight, more preferably 1 to 30 % by weight, most preferably 1 to 20 % by weight, specifically 1 to 10 % by weight and most specifically 1 to 6 % by weight of amorphous cellulose based on the weight of the beads.

Cellulose is a polysaccharide consisting of long unbranched chains of β-1,4- linked D-glucopyranose units. As described by Parks et al. (Biotechnology for Biofuels, 2010, 3:10 p. 3 to 10: Cellulose crystallinity index: Measurement techniques and their impact on interpreting cellulase performance. Sunkyu Park, John O. Baker, Michael E. Himmel, Philip A. Parilla and David K. Johnson) there are four types of cellulose with different crystallinity index.

For the invention different structures of cellulose are suitable, such as cellulose type I, a cellulose structure present in plants which is not changed by the recovery or cellulose type II, a thermodynamic more stable structure, which is prepared by dissolving type I cellulose and regenerating the cellulose.

Thus for the manufacturing of the beads various types of cellulose independent of their degree of crystallinity can be used as the cellulose is completely dissolved in a suitable solvent during the manufacturing process. The amount of lignin in the used cellulose should not be high due to its poor solubility, but commercially available cellulose types for cosmetic or pharmaceutical use can be used. Microcrystalline cellulose is preferred due to its purity and relatively low molecular weight.

The amount of cellulose must be adapted according to the intended use. More solid beads with higher peeling and scrubbing properties have a higher amount of cellulose, more softer beads which are used to apply the emollients on the skin comprise a low amount of cellulose. Examples of suitable bead compositions are:

Solid beads are comprising:

75 - 80 % by weight amorphous cellulose and
20 - 25 % y weight of a cosmetic ingredient and
less than 10 % by weight of water
based on the weight of the beads.

Soft beads are comprising

1 - 20 % by weight amorphous cellulose and
80 - 99 % by weight of a cosmetic ingredient and
less than 3 % by weight of water based on the weight of the beads and more preferably
1 - 10 % by weight amorphous cellulose and
90- 99 % by weight of a cosmetic ingredient and
less than 1 % by weight of water
based on the weight of the beads.

Solid beads have more abrasive properties while the soft beads are advantageous for the application of cosmetic oils.

In the prefered embodiment of the invention the beads do not comprise cellulose derivatives. Biodegradability of the beads decreases if derivatized cellulose is used. This means that the cellulose which is used for manufacturing the beads is not functionalized by etherification, esterification, oxidation, grafting or any other derivatization because of the superior compatibility of pure cellulose in cosmetic or pharmaceutic applications and due to the biodegradability of the beads.

**Cosmetic Ingredients**

Cosmetic ingredients which are incorporated in the beads of the invention for at least 20 % by weight of the beads encompass auxiliaries and additives selected from the group consisting of surfactants, oil components, emollients, emulsifiers, pearlescent waxes, consistency regulators, thickeners, superfatting agents, stabilizers, polymers, silicone compounds, fats, waxes, lecithins, phospholipids, vitamins, provitamins, enzymes, melanin, carotenoids, UV photoprotective factors, biogenic active ingredients, biological and plant extracts, antioxidants, deodorants, repellents, antiperspirants, antidandruff agents, antiacne agents, film formers, swelling agents, insect repellents, self-tanning agents, skin-whiteners, hydrotropes, solubilizers, preservatives, perfume oils, fragrances, dyes and mixtures thereof. The preferred cosmetic ingredients incorporated in the beads are selected from the group consisting of emollients, oil components, superfatting agents, waxes and mixtures thereof, most preferred are emollients.

The incorporated cosmetic ingredients have a molecular weight of more than 50 g/mol, preferably more than 100 g/mol. Incorporation of ingredients with lower molecular weight has shown to have limited use in cosmetic formulations.

**Emollients**

In a prefered embodiment of the invention the incorporated cosmetic ingredients are emollients. These are selected from the group consisting of fatty acid esters, esters of C6-C28 fatty acids and C6-C28 fatty alcohols, glyceryl esters, fatty acid ester ethoxylates, alkyl ethoxylates, C12-C28 fatty alcohols, C12-C28 fatty acids, Guerbet esters, Guerbet alcohols and Guerbet acids, saturated alkanes, C12-C28 fatty alcohol ethers, vegetable oils, natural essential oils, mineral oil, parafinum liquidum, petrolatum, isoparaffins, preferably the emollients are selected from the group consisting of dibutyl adipate (Cetiol® B), phenethyl benzoate, coco-caprylate (Cetiol® C5), coco-caprylate/caprate (Cetiol® LC, Cetiol® C5, Cetiol® C 5C), dicaprylyl Carbonate (Cetiol® CC), propylheptyl caprylate (Cetiol® Sensoft), caprylyl caprylate/caprate (Cetiol® RLF), myristyl myristate (Cetiol® MM), PEG-7 caprylic/capric glycerides (Cetiol® HE810), isononyl isononanoate (Cetiol® ININ) ; capric glycerides, coco-glycerides (Myritol® 331), capryl/caprin-triglyceride (Myritol® 312), capryl/caprin-triglyceride (Myritol® 318), C12-15 alkyl benzoate (Cetiol® AB), PPG-3 benzyl ether myristate, C12-13 alkyl lactate, isodecyl salicylate, alkyl malate, isoamyl laurate, propylheptyl caprylate, butyloctyl salicylate, polycrylene, dicaprylyl carbonate (Cetiol® CC), dicaprylyl ether (Cetiol® OE), 2-octyldodecylmyristate, isohexadecane, dimethyl capramide, squalene, isopropyl isostearate, isostearyl isostearate, decyl oleate (Cetiol® V), oleyl erucate (Cetiol® J 600), ethylhexyl stearate (Cetiol® 868); cetearyl ethylhexanoate (Luvitol® EHO), octyldodecanol (Eutanol® G), hexyldecanol (Eutanol® G16), volatile linear C8 to C16 alkanes, C10 to C15 alkanes, C11-C13 alkanes (Cetiol® Ultimate), C13-15 alkanes, C15-19 alkanes, C17-23 alkanes, isododecane, undecane, tridecane (Cetiol® Ultimate), dodecane, propylene glycol dipelargonate, diisopropyl sebacate, cetearyl isononanoate (Cetiol® SN), isononyl isononanoate, isocetyl stearoyl stearate, dipentaerithrityl hexacaprylate/hexacaprate, isodecyl neopentanoate, PEG-6 caprylic/ capric glycerides (Cetiol® 767), caprylic/capric triglyceride (Myritol® 312, Myritol® 318), ethylhexyl stearate, ethylhexylcocoate, ethylhexyl stearate (Cetiol® 868), dipropylheptyl carbonate (Cetiol® 4 All), hexyl laurate (Cetiol® A), dicaprylyl carbonate, PEG-7 glyceryl cocoate (Cetiol® HE), polyglyceryl-3 diisostearate (Lameform® TGI), lauryl alcohol, methyl canolate (Cetiol® MC), hexyldecyllaurate and hexyldecanol (Cetiol® PGL), hexyldecyl stearate (Eutanol® G 16S), PPG-15 Stearylether (CETIOL® E), ethylhexyl palmitat (CEGESOFT® C24), pentaerythrityl tetraethylhexanoate (Cetiol® PEEH4), hexyldecyllaurate and hexyldecanol (Cetiol® PGL), caprylyl caprylate/ caprate (Cetiol® RLF).

Further suitable oil components which can be used as emollients and incorporated in the beads are natural products selected from the group consisting of Elaeis guiineensis oil (Cegesoft® GPO), Passiflora incarnata seed oil (Cegesoft® PFO), olive oil, olus oil (Cegesoft® PS6), Butyrospermum parkii butter (Cetiol® SB 45), ethylhexylcocoat (and) Cocos Nucifera Öl (CETIOL® COCO), Shorea stenoptera seed butter (Cegesoft® SH), almond oil, avocado oil, borage oil, canola oil, castor oil, chamomile, coconut oil, corn oil, cottonseed oil, jojoba oil, evening primrose oil, papaya oil, palm oil, hazelnut oil, peanut oil, walnut oil, safflower oil, sesame oil, soybean oil, sunflower oil, sweet almond, a rice bran/wheat germ oil, rosehip oil, Ricinus communis oil, lanolin; hydrogenated vegetable oil, Candelilla cera, Euphorbia vegetable oil (Cegesoft® VP), sterols and derivatives.

In addition silicones and silicone derivatives such as polydimethylsiloxanes, methicone, dimethicone, cyclomethicone, caprylyl methicone, dimethicone copolyol, undecylcrylene dimethicone, dimethiconol, trimethicone, organosiloxanes are used as oil components in these loaded beads.

More preferably the emollients are selected from the group consisting of dibutyl adipate, caprylic/capric triglyceride, coco-caprylate/caprate, caprylyl caprylate, capric glycerides, coco-glycerides, C12-15 alkyl benzoate, propylheptyl caprylate, dicaprylyl carbonate, dicaprylyl ether, isohexadecane, isopropyl isostearate, octyldodecanol, volatile linear C8 to C16 alkanes, C10 to C15 alkanes, C11-C13 alkanes, C13-15 alkanes, C15-19 alkanes, isononyl isononanoate, PEG-6 Caprylic/ Capric Glycerides, Ethylhexyl Stearate, Dipropylheptyl Carbonate, Hexyl Laurate, Dicaprylyl Carbonate, Oleyl Erucate, PEG-7 Glyceryl Cocoate, Lauryl Alcohol, Methyl Canolate, Myristyl Myristate, Hexyldecyllaurate. Hexyldecanol, Cetearyl Isononanoate, Hexyldecyl Stearate, Polyglyceryl-3 Diisostearate, natural essential oils, mineral oil, parafinum liquidum, isododecane, undecane, dodecane, tridecane.

Most preferably the emollients are selected from the group consisting of dibutyl adipate, caprylic/capric triglyceride, coco-caprylate/caprate, caprylyl caprylate, capric glycerides, coco-glycerides, C12-15 alkyl benzoate, propylheptyl caprylate, dicaprylyl carbonate, dicaprylyl ether, isohexadecane, isopropyl isostearate, octyldodecanol, volatile linear C8 to C16 alkanes, isododecane, undecane, dodecane, tridecane, isononyl isononanoate, PEG-6 Caprylic/ Capric Glycerides, Ethylhexyl Stearate, Dipropylheptyl Carbonate, Hexyl Laurate, Dicaprylyl Carbonate, Oleyl Erucate, PEG-7 Glyceryl Cocoate, Lauryl Alcohol, Methyl Canolate, Myristyl Myristate, Hexyldecyllaurate. Hexyldecanol, Cetearyl Isononanoate, Hexyldecyl Stearate, Polyglyceryl-3 Diisostearate.

In a specific embodiment of the invention the cosmetic ingredients are emollients selected from the group consisting of undecane, dodecane, tridecane, dibutyl adipate, dicaprylyl carbonate, dicaprylyl ether, propyl heptyl caprylate or mixtures thereof. These are preferred emollients which are often used as a substitute for silicones and silicone derivatives as there is an increasing demand to avoid the latter in cosmetic and personal care compositions for ecological reasons.

Preferably the cosmetic ingredients have a melting point below 80°C, preferably the melting point is below 50°C and more preferably the melting point is below 30°C, most preferably the cosmetic ingredients are liquid at room temperature (20 to 25°C) .

Preferably the cosmetic ingredients have a boiling point above 100°C at 1 atmosphere of pressure (sea level), preferably the boiling point is above 130°C, more preferably the boiling point is above 150°C and most preferably the boiling point is above 180°C at 1 atmosphere of pressure (sea level). The higher the boiling point of the incorporated ingredient is, the lower the shrinkage during storage of the beads is.

## Cosmetic compositions

Another embodiment of the invention is the use of the beads as peeling agent or for application of cosmetic ingredients, especially emollients on the skin.

The beads of the present invention can be incorporated into aqueous, semi-aqueous or nonaqueous cosmetic compositions. Cosmetic compositions shall mean any composition which is applied in contact with the various external parts of the human body (epidermis, hair system, nails, lips) or with the teeth and the mucous membranes of the oral cavity with the intend to cleaning them, perfuming them, changing their appearance and/or correcting body odours and/or protecting them or keeping them in good condition.

The cosmetic compositions according to the invention can for example be in the form of a hair shampoos, hair lotions, foam baths, shower baths, creams, gels, lotions, alcoholic and aqueous/alcoholic solutions, foams, emulsions, wax/fat masses, stick preparations, powders or ointments.

The cosmetic compositions comprise the spherical beads according to the invention in an amount of 0.1 to 50 weight-% based on the total weight of the composition, preferably in an amount of 1 to 30, especially in an amount of 2 to 10 weight-% based on the total weight of the composition.

Depending on their intended application, the cosmetic formulations contain a number of other auxiliaries and additives such as, for example, surface-active substances (surfactants, emulsifiers), other oil components, pearlizing waxes, consistency factors, thickeners, superfatting agents, stabilizers, polymers, fats, waxes, lecithins, phospholipids, biogenic agents, UV protection factors, antioxidants, deodorizers, antiperspirants, antidandruff agents, film formers, swelling agents, insect repellents, self-tanning agents, tyrosinase inhibitors (depigmenting agents), hydrotropes, solubilizers, preservatives, perfume oils, dyes and the like which are listed in the following.

The quantities in which the particular additives are used are determined by the intended application.

## Process for Manufacturing Cellulose Beads

For the preparation of cellulose beads basically a solution of dissolved cellulose is prepared which could then be utilized to form cellulose beads by different techniques such as dropping, spraying, emulsification by $\mu$-fluidics or $\mu$-jet reactor.

The preferred manufacturing method is a three-step process comprising: 1. the dissolution, 2. precipitation and 3. loading, optionally a drying step can be added.

Manufacturing cellulose beads via **dropping** comprises the following steps:

1. Dissolution: A solution of cellulose at a concentration of 0.5 to 20 %, preferably 1 to 15 % by weight in an organic electrolyte solution comprising an organic solvent (52 - 88 by weight %; e.g. acetone, butanone, acetonitrile or acrylonitrile) and a quaternary ammonium salt (8 - 35 by weight %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of European application WO2019/081246. Optionally active ingredients (e.g. liquids, solids, dyes, polymers, enzymes) can be introduced to the solution by either dissolution or dispersion in the cellulose solution.

2. Droplet Formation/Precipitation: The solution is then dropped into a precipitation bath of a protic solvent or into a precipitation bath of mixtures of solvents, where one of the solvents is protic, preferably selected from the group consisting of water, ethanol, methanol, propanol, isopropanol, butanol, glycerin, solutions of organic or inorganic salts, buffer solution, acetonitrile/ethanol 90:10; dichloromethane/ethanol 90:10; water/acetone 50:50 and mixtures thereof. More preferably the solvent of the precipitation bath is selected from the group consisting of ethanol or propanol, most preferably ethanol.

The droplet formation occurs in the gas phase 0.1 to 100 cm, preferably 0.5 to 30 cm, above the surface of the precipitation bath (air, nitrogen, argon, or vapor of the precipitation bath) by passing the solution through an open end canula, tube, pipette, nozzle or any other system (i.e. vibrating nozzle) to form droplets. Upon contact with the liquid phase of the precipitation bath, the droplets solidify instantaneously and remain in droplet form as individual separate particles. The resulting beads may directly sink to the bottom of the precipitation bath (example: ethanol, methanol) or stay at the surface (example: dichloromethane/ethanol) or stay at the surface of the precipitation bath at first and sink to the bottom when fully equilibrated with precipitation medium (example: water), depending on the density of the precipitation medium.

3. Isolation/Filtration and Loading: The resulting soaked (with precipitation medium) beads are isolated by filtration and may be re-dispersed in a fresh precipitation bath. This washing procedure can be repeated several times until no residuals of the organic electrolyte solution can be detected by elemental analysis. The isolated beads soaked with precipitation medium can be either dried (air, vacuum, heat) or dispersed in an active ingredient (oils and emollients, salt solutions, polymer solutions, enzyme solutions, dyes and pigments) or solutions thereof for loading. Upon complete equilibration the beads may be isolated by filtration.

Manufacturing cellulose beads via **spraying** comprises the following steps:

1. Dissolution: A solution of cellulose at a concentration of 1.0 to 10 %, preferably 2 to 5 % by weight in an organic electrolyte solution comprising an organic solvent (52 - 88 %; e.g. acetone, butanone, acetonitrile or acrylonitrile) and a quaternary ammonium salt (8 - 35 %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of WO2019/081246. Optionally active ingredients (e.g. liquids, solids, polymers, enzymes) can be introduced to the solution by either dissolution or dispersion in the cellulose solution.

2. Precipitation: The solution is then sprayed into a precipitation bath of a protic solvent or into a precipitation bath of mixtures of solvents, where one of the solvents is protic, preferably selected from the group consisting of water, ethanol, methanol, propanol, butanol, glycerin, solutions of organic or inorganic salts, buffer solution, acetonitrile/ethanol 90:10; dichloromethane/ethanol 90:10; water/acetone 50:50 and mixtures thereof. More preferably the solvent of the precipitation bath is selected from the group consisting of ethanol or propanol, most preferably ethanol. The droplet formation occurs in the gas phase 0.1 to 100 cm, preferably 0.5 to 30 cm, above the surface of the precipitation bath (air, nitrogen, argon, or vapor of the precipitation bath) by passing the cellulose solution through a nozzle with high pressure. Upon contact with the liquid phase of the precipitation bath, the droplets solidify instantaneously and remain in droplet form as individual separate particles. The resulting beads directly sink to the bottom of the precipitation bath (ethanol, methanol, etc.).

3. Isolation/Filtration: The resulting soaked (with precipitation medium) beads are isolated by filtration and may be redispersed in a fresh precipitation bath. This washing procedure can be repeated several times until no residuals of the organic electrolyte solution can be detected by elemental analysis. The isolated beads soaked with precipitation medium can be either dried (air, vacuum, heat) or dispersed in an active ingredient (oils and emollients, salt solutions, polymer solutions, enzyme solutions) or solutions thereof for loading. Upon complete equilibration the beads may be isolated be filtration. The bead size was measured by laser diffraction and optical microscopy.

Manufacturing cellulose beads via **emulsification** comprising the following steps:

1. Dissolution: A solution of cellulose at a concentration of 1.0 to 10 %, preferably 2 to 5 % by weight in an organic electrolyte solution comprising an organic solvent (52 - 88 %; e.g. acetone, butanone, acetonitrile or acrylonitrile) and a quaternary ammonium salt (8 - 35 %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of WO2019/081246. Optionally active ingredients (e.g. liquids, solids, polymers, enzymes) can be introduced to the solution by either dissolution or dispersion in the cellulose solution.

2. Emulsification: The cellulose solution is emulsified in an oil phase consisting of a predominantly hydrophobic oil such as for example an emollient or plant oil and a surfactant in a ratio of cellulose solution to oil phase of 1:1 to 1:10, preferably 1:1 to 1:5 more preferably 1:2 to 1:3 using an Ultra Turrax@ at 10000 rpm for 30 s. Alternatively the emulsification can be conducted with a μ-jet reactor, via μ-Fluidics or any other instrument available for emulsification.

3. Precipitation: The emulsion is then dropwise added to a precipitation bath of a protic solvent or into a precipitation bath of mixtures of solvents, where one of the solvents is protic, preferably selected from the group consisting of H2O, ethanol, methanol, propanol, butanol, glycerin, solutions of organic or inorganic salts, buffer solution, acetonitrile/ethanol 90:10; dichloromethane/ethanol 90:10; water/acetone 50:50 and mixtures thereof. More preferably the solvent of the precipitation bath is selected from the group consisting of ethanol or propanol, most preferably ethanol. Upon contact with the liquid phase of the precipitation bath, the droplets solidify instantaneously and remain in droplet form as individual separate particles.

4. Isolation/Filtration: The resulting soaked (with precipitation medium) beads are isolated by centrifugation (3500 rpm, 10 min) and may be redispersed in a fresh precipitation bath. This washing procedure can be repeated several times until no residuals of the organic electrolyte solution can be detected. The isolated beads soaked with precipitation medium can be either dried (air, vacuum, heat) or dispersed in an active ingredient (oils and emollients, salt solutions, polymer solutions, enzyme solutions) or solutions thereof for loading. Upon complete equilibration the beads may be isolated be filtration. The bead size was measured by laser diffraction.

**Specific examples for preparing cellulose beads**

Raw materials

Cellulose:

In general, all types of cellulose materials with a predominant amount of cellulose can be used for this process. The process parameters for dissolution (time, viscosity) may vary for cellulose materials of different molecular weight.

The cellulose used in the current examples was microcrystalline cellulose for short referred to as MCC - microcrystalline cellulose (Source: Sigma-Aldrich, characteristics: degree of crystallinity according to solide state 13C-NMR: 57 %, see below.)

Following quaternary ammonium compounds (tetra-butyl-ammonium - TBA-) have been used:

Tetra-butyl-ammonium-acetate, (TBA-Acetate)
Tetra-butyl-ammonium-acrylate, (TBA-Acrylate, TBAAc)
Tri-butyl-methyl-ammonium-acetate, (TBMA-Acetate)
Allyl-tri-butyl-ammonium-acetate, (ATBA-Acetate)

TBA-Acetate and TBA-Acrylate have been prepared via anion exchange from TBA-chloride obtained from Sigma Aldrich. TBMA-Acetate has been prepared via anion exchange from TBMA-chloride obtained from Sigma Aldrich.

Emollients: Cetiol®-types by BASF Personal Care and Nutrition GmbH, Germany

Cetiol®B - Dibutyl Adipate
Cetiol® CC - Dicaprylyl Carbonate
Cetiol® OE - Dicaprylyl Ether
Cetiol® Ultimate - Undecane, Tridecane

Dissolution of cellulose

**Examples for the dissolution of cellulose (summarized in table 1):**

Procedure for the dissolution of cellulose in TBAAc / MeCN (Ex. 1.1 - Ex. 1.5 and Ex. 1.9)

Cellulose (29.5 g) was added to a solution of tetrabutylammonium acetate (95.3 g) in acetonitrile (160 g) and stirred for 30 min at ambient temperature. The temperature was increased to 65°C and the solvent was removed under reduced pressure. Acetonitrile (300 g) was dosed to the vessel over a period of 9 h at a temperature of 65°C to obtain a clear, colorless solution. The obtained solution (424.8 g) with a composition of cellulose / TBAAc / acetonitrile (6.9 / 22.4 / 70.6 wt%) was further diluted with acetonitrile and TBAAc to yield the desired composition.

Procedure for the dissolution of cellulose in TBAAc / MeCN (Ex. 1.6 - Ex. 1.8 and Ex 1.10 - Ex. 1.18)

Cellulose (3.0 - 13.0 wt%) was added to a solution of tetrabutylammonium acetate (8.4 - 36.5 wt%) in acetonitrile (100 - 300 mL) and stirred for 30 min at ambient temperature. The temperature was increased to 65°C and the solvent was removed under reduced pressure. Acetonitrile (50.1 - 88.6 wt%) was dosed to the vessel over a period of 9 h at a temperature of 65°C to obtain a clear, colorless solution.

Procedure for the dissolution of cellulose in TBAC/acetone (Ex. 1.19 and Ex. 1.20)

70.0 g of TBAC, 7.0 g of MCC and 35.0 g of acetone were added to a 250 mL four-necked flask. The mixture was stirred at 50 °C for 10 min. The volatiles were removed at 125 °C external temperature. After cooling to 85 °C, the viscous melt was stirred for 15 h. The desired amount of acetone was added to the clear, orange melt in 10 min, which was cooled down to 65 °C. The final solution was stirred at 65 °C for 30 min.

Table 1: Examples of cellulose solutions for the preparation of beads.

| Example | QUAT | Solvent | Cellulose (wt%) | QUAT (wt%) | Solvent (wt%) |
|---|---|---|---|---|---|
| Ex. 1.1 | TBAAc | MeCN | 0.5 | 5.0 | 94.5 |
| Ex. 1.2 | TBAAc | MeCN | 0.5 | 14.6 | 84.9 |
| Ex. 1.3 | TBAAc | MeCN | 1.0 | 14.4 | 84.6 |
| Ex.1.4 | TBAAc | MeCN | 2.5 | 20.6 | 76.9 |
| Ex. 1.5 | TBAAc | MeCN | 2.5 | 28.4 | 69.1 |
| Ex. 1.6 | TBAAc | MeCN | 3.0 | 8.4 | 88.6 |
| Ex. 1.7 | TBAAc | MeCN | 3.0 | 14.2 | 82.8 |
| Ex. 1.8 | TBAAc | MeCN | 5.0 | 13.9 | 81.1 |
| Ex. 1.9 | TBAAc | MeCN | 5.0 | 14.3 | 80.7 |
| Ex. 1.10 | TBAAc | MeCN | 5.0 | 17.5 | 77.5 |
| Ex. 1.11 | TBAAc | MeCN | 5.0 | 27.4 | 67.6 |
| Ex. 1.12 | TBAAc | MeCN | 6.0 | 26.3 | 67.7 |
| Ex. 1.13 | TBAAc | MeCN | 6.25 | 28.3 | 65.4 |
| Ex. 1.14 | TBAAc | MeCN | 6.9 | 22.4 | 70.6 |
| Ex. 1.15 | TBAAc | MeCN | 10.0 | 27.8 | 62.2 |
| Ex. 1.16 | TBAAc | MeCN | 10.0 | 33.4 | 56.6 |
| Ex. 1.17 | TBAAc | MeCN | 10.9 | 36.5 | 52.6 |
| Ex. 1.18 | TBAAc | MeCN | 13.0 | 36.1 | 50.9 |
| Ex. 1.19 | TBAC | Acetone | 3.2 | 32.3 | 64.5 |
| Ex. 1.20 | TBAC | Acetone | 4.6 | 44.6 | 50.8 |

**Examples for the preparation of pure cellulose beads (summarized in table 2)**

Equipment: 3 l beaker with PTFE half-moon-blade stirrer and dropping funnel

The beaker was charged with 500 g of ethanol. With a rate of 1 drop per second, 10.0 g of cellulose solution (Ex. 1.1 - Ex. 1.20) were dropped from a height of 6 cm into the stirred ethanol. The stirrer was stopped after completion of the feed. The obtained cellulose beads sink to the bottom of the breaker immediately upon addition to the ethanol. The cellulose beads were isolated by filtration. The cellulose beads were suspended in 2000 ml fresh ethanol for 1 h and isolated by filtration. This washing step including suspention and filtration was repeated one more time. The resulting isolated beads were dried in vacuum (40°C, 2 h, 100 mbar).

In order to verify that the washing procedure was sufficiently repeated until no residuals of the organic electrolyte solution could be detected an elemental analysis of the cellulose beads of example 2.3 was conducted, whereby the microcrystalline cellulose (MCC) was subjected to the same washing steps as the beads. The results for the MCC were: Carbon 43.5 g/100 g, Oxygen 50 g/100 g, Nitrogen < 0.5 g /100 g, Hydrogen 6.7 g /100 g, the cellulose beads resulted in Carbon 43.4 g/100 g, Oxygen 50 g/100 g, Nitrogen < 0.5 g /100 g,

Table 2: Examples of pure cellulose beads. Manufactured for $\mu$-CT :

| Example | Solution Number | Cellulose concentration of solution (wt.%) | Cellulose content in beads (wt.%) | Water content in beads (wt.%) | Bead size [mm] |
|---|---|---|---|---|---|
| Ex. 2.1 | Ex. 1.17 | 10.9 | > 99.5 | < 0.5 | 1.90 |
| Ex. 2.2 | Ex. 1.16 | 10.0 | > 99.5 | < 0.5 | 1.05 |

(continued)

| Example | Solution Number | Cellulose concentration of solution (wt.%) | Cellulose content in beads (wt.%) | Water content in beads (wt.%) | Bead size [mm] |
|---|---|---|---|---|---|
| **Ex. 2.3** | Ex. 1.9 | 5.0 | > 99.5 | < 0.5 | 0.84 |

**Examples for the preparation of cellulose beads containing cosmetic ingredients - inventive examples (summarized in table 3)**

Beads produced from TBAAc/MeCN loaded with Cetiol types (Ex. 3.1 - Ex. 3.4 and Ex. 3.12 - Ex. 3.17):

Equipment: 3 l beaker with PTFE half-moon-blade stirrer and three diaphragm pump SIMDOS 02 from KNF.

The beaker was charged with 2000 ml of ethanol. With a rate of 3 mL/min, 193.6 g of cellulose solution in **TBAAc/MeCN** (Ex. 1.1 - Ex. 1.18) were dropped from a height of 0.5 cm through a canula with a diameter of 0.95 mm into the stirred ethanol. The stirrer was stopped after completion of the feed. The obtained cellulose beads sink to the bottom of the breaker immediately upon addition to the ethanol. 1 h after completion of the feed, the cellulose beads were isolated by filtration. The cellulose beads were suspended in 2000 ml fresh ethanol for 1 h and isolated by filtration. This washing step including suspension and filtration was repeated one more time.

69.1 g of isolated beads were added to a mixture of 251.3 g of Cetiol type (see table Z) and 249.8 g of ethanol in a 1 L glass beaker. After 1 h the cellulose beads were isolated by filtration and added 502.6 g of fresh Cetiol. This step was repeated with 507.1 g of Cetiol without ethanol. The open vial with Cetiol and cellulose beads were stored for 2 days in a vacuum cabinet at 50 mbar and ambient temperature. The cellulose beads were isolated by filtration. The weight of the isolated beads was 62.4 g. The beads were stored in a closed container at ambient conditions under air atmosphere.

Beads produced from **TBAC/acetone** loaded with Cetiol types (Ex. 3.6 - Ex. 3.9):

Equipment: 3 L beaker with PTFE half-moon-blade stirrer and three diaphragm pump SIMDOS 02 from KNF.

The beaker was charged with 2000 mL of ethanol. With a rate of 3 mL/min, 193.6 g of cellulose solution in TBAC/acetone (Ex. 1.19) were dropped from a height of 0.5 cm through a canula with a diameter of 0.95 mm into the stirred ethanol. The stirrer was stopped after completion of the feed. The obtained cellulose beads sink to the bottom of the breaker immediately upon addition to the ethanol. 1 h after completion of the feed, the cellulose beads were isolated by filtration. The cellulose beads were suspended in 2000 mL fresh ethanol for 1 h. This washing step was repeated.

69.1 g of isolated beads were added to a mixture of 251.3 g of Cetiol (see table Z) and 249.8 g of ethanol in a 1 L glass beaker. After 1 h the cellulose beads were isolated by filtration and added 502.6 g of fresh Cetiol. This step was repeated with 507.1 g of Cetiol. The open vial with Cetiol and cellulose beads were stored for 2 days in a vacuum cabinet at 50 mbar and ambient temperature. The cellulose beads were isolated by filtration. The weight of the isolated beads was 62.4 g. The beads were stored in a closed container at ambient conditions under air atmosphere.

**Beads loaded with a mint mixture (Ex. 3.10):**

Equipment: 3 L beaker with PTFE half-moon-blade stirrer and dropping funnel

The beaker was charged with 1000 g of ethanol. With a rate of 1 mL/min, 100.0 g of cellulose solution in TBAAc/MeCN (Ex. 1.4) were dropped from a height of 6 cm through a canula with a diameter of 0.95 mm into the stirred ethanol. The stirrer was stopped after completion of the feed. The obtained cellulose beads sink to the bottom of the breaker immediately upon addition to the ethanol. The cellulose capsules were isolated by filtration. The weight of the isolated wet beads was 27.1 g and the solid content was 11.9 wt.%. The capsule diameter d50 was 2.4 mm. 10 g of the wet capsules were dispersed in 50 g of a mixture of L-Isopulegol (23.5 g), L-Menthylacetat (32.0 g), L-menthon (365 g) and L-Menthol (582.5 g) for 24 h. 10.7 g of wet beads with a solid content of 9.73 g with a diameter of d50 of 2.4 mm (determined by light microscopy) could be isolated by filtration.

**Beads loaded with a perfume mixture (Ex. 3.11):**

Equipment: 50 mL vial and dropping funnel

The vial was charged with 20 g of ethanol. With a rate of 1 mL/min, 1.0 g of cellulose solution in TBAAc/MeCN (Ex. 1.17) were dropped from a height of 6 cm through a canula with a diameter of 0.95 mm into the stirred ethanol. The stirrer was stopped after completion of the feed. The obtained cellulose beads sink to the bottom of the vial immediately upon addition to the ethanol. The cellulose beads were isolated by filtration. The wet beads were dispersed in 10 g of a perfume mixture (Perfurme Oil COWBOY Super 719F, BASF) for 24 h and isolated by filtration.

Table 3: Examples for beads containing cosmetic ingredients.

| Examples | Cellulose solution | Bead size [mm] | Active ingredient | Theoretical active concentration [%] | Experimental active concentration [%] |
|---|---|---|---|---|---|
| Ex. 3.1 | Ex. 1.4 | 2.0 | Cetiol OE | 97.5 | 95.8 |
| Ex. 3.2 | Ex. 1.4 | 2.0 | Cetiol B | 97.5 | 96.5 |
| Ex. 3.3 | Ex. 1.4 | 2.0 | Cetiol CC | 97.5 | 96.0 |
| Ex. 3.4 | Ex. 1.4 | 2.0 | Cetiol Ultimate | 97.5 | 95.5 |
| Ex. 3.5 | Ex. 1.4 | 0.5 | Cetiol OE | 97.5 | |
| Ex. 3.6 | Ex. 1.20 | 2.0 | Cetiol OE | 95.4 | 92.3 |
| Ex. 3.7 | Ex. 1.20 | 2.0 | Cetiol Ultimate | 95.4 | 91.7 |
| Ex. 3.8 | Ex. 1.20 | 2.0 | Cetiol CC | 95.4 | 93.5 |
| Ex. 3.9 | Ex. 1.20 | 2.0 | Cetiol B | 95.4 | 94.4 |
| Ex. 3.10 | Ex. 1.4 | 2.4 | Mint mix | 75.0 | |
| Ex. 3.11 | Ex. 1.20 | 2.0 | Perfume | 95.4 | |
| Ex. 3.12 | Ex. 1.5 | 1.1 | Cetiol OE | 97.5 | 97.2 |
| Ex. 3.13 | Ex. 1.13 | 1.4 | Cetiol OE | 93.75 | 90.5 |
| Ex. 3.14 | Ex. 1.13 | 1.9 | Cetiol OE | 93.75 | 89.7 |
| Ex. 3.15 | Ex. 1.16 | 2.3 | Cetiol OE | 90.0 | 81.7 |
| Ex. 3.16 | Ex. 1.16 | 2.1 | Cetiol OE | 90.0 | 84.0 |
| Ex. 3.17 | Ex. 1.11 | 1.7 | Cetiol OE | 95.0 | 92.2 |

Table 4: Pressure profile measurements

| Beads from example | Active content (Cetiol OE) [wt.%] | Cellulose content [wt.%] | Cellulose in solution [wt.%] | Average Diameter D (4,3) [μm] | Average Force 20% Way [N] | Average Force 50% Way [N] | Average Force 80% Way [N] |
|---|---|---|---|---|---|---|---|
| Comparative material "Vivapor CS 800 S" | 0.0 | 100 | | 932 | 32.5 | 36.6 | 73.4 |
| Comparative Ex. 2.2 | 0,0 | 100 | 10.00 | 1053 | 42.8 | 100.0 | |
| Ex. 3.12 | 97.2 | 2.8 | 2.50 | 1067 | 0.2 | 1.0 | 3.5 |

(continued)

| Beads from example | Active content (CetiolOE) [wt.%] | Cellulose content [wt.%] | Cellulose in solution [wt.%] | Average Diameter D (4,3) [μm] | Average Force 20% Way [N] | Average Force 50% Way [N] | Average Force 80% Way [N] |
|---|---|---|---|---|---|---|---|
| Ex. 3.13 | 90.5 | 9.5 | 6.25 | 1412 | 2.9 | 9.3 | 22.3 |
| Ex. 3.14 | 89.7 | 10.3 | 6.25 | 1891 | 5.2 | 18.0 | 37.4 |
| Ex. 3.15 | 81.7 | 18.3 | 10.00 | 2289 | 19.9 | 57.6 | 137.1 |
| Ex. 3.16 | 84.0 | 16.0 | 10.00 | 2070 | 14.8 | 48.9 | 120.4 |
| Ex. 3.17 | 92.2 | 7.8 | 5.00 | 1701 | 2.4 | 9.8 | 21.7 |

**Claims**

1. Spherical beads with a diameter D(4,3) of 1 - 5000 μm comprising
1 to 80 % by weight of amorphous cellulose,
at least 20 % by weight of a cosmetic ingredient with a molecular weight > 50 g/mol, and less than 10 % by weight of water based on the weight of the beads.

2. Beads according to claim 1, **characterized in that** the cosmetic ingredient is selected from the group consisting of surfactants, oils, emollients, emulsifiers, pearlescent waxes, consistency regulators, thickeners, superfatting agents, stabilizers, polymers, silicone compounds, fats, waxes, lecithins, phospholipids, vitamins, provitamins, enzymes, melanin, carotenoids, UV photoprotective factors, biogenic active ingredients, biological and plant extracts, antioxidants, deodorants, repellents, antiperspirants, antidandruff agents, antiacne agents, film formers, swelling agents, insect repellents, self-tanning agents, skin-whiteners, hydrotropes, solubilizers, preservatives, perfume oils, fragrances, dyes and mixtures thereof.

3. Beads according to claim 1 or 2, **characterized in that** the cosmetic ingredients are selected from the group consisting of emollients, oils, superfatting agents, waxes and mixtures thereof.

4. Beads according to one of claims 1 to 3, **characterized in that** the cosmetic ingredient has a melting point below 80°C.

5. Beads according to one of claims 1 to 4, **characterized in that** the cosmetic ingredient has a boiling point above 100°C at 1 atmosphere of pressure (sea level).

6. Beads according to one of claims 1 to 5, **characterized in that** they comprise
less than 5 % , preferably less than 3 % and more preferably less than 1 % by weight of water based on the weight of the beads.

7. Beads according to one of claims 1 to 6, **characterized in that** they comprise
at least 35 % by weight, preferably at least 60 % by weight and more preferably at least 70 % by weight, even more preferably 80 % by weight and most preferably at least 95 % by weight of the cosmetic ingredient based on the weight of the beads.

8. Beads according to one of claims 1 to 7, **characterized in that** they comprise
1 to 50 % by weight preferably 1 to 30 % by weight, more preferably 1 to 20 % by weight, even more preferably 1 to 10 % and specifically 1 to 6 % by weight of amorphous cellulose based on the weight of the beads.

9. Beads according to one of claims 1 to 8, **characterized in that** they are translucent.

10. Beads according to one of claims 1 to 9, **characterized in that** their deformation between two parallel plates obeys to the rules

$F_{50} < 0.03 \times D -14.95$ for beads of D(4,3) of 500 to 1500 μm and

$F_{50} < 0.12 \times D -149.95$ for beads of D(4,3) of 1500 to 3000 $\mu$m

with $F_{50}$ being the force measured in N that is necessary to compress the bead to 50% of its original diameter D measured in $\mu$m and wherein $F_{50}$ is larger than 0.05 N.

11. Beads according to one of claims 1 to 10, **characterized in that** they have a diameter D(4,3) of 100 to 4000 $\mu$m, preferably 500 to 3000 $\mu$m, more preferably 1000 to 3000 $\mu$m and most preferably 2000 to 3000 $\mu$m determined by light microscopy.

12. Beads according to one of claims 1 to 6 or 9 to 11, **characterized in that** they comprise
   75 - 80 % by weight amorphous cellulose and
   20 - 25 % by weight of a cosmetic ingredient and
   less than 10 % by weight of water based on the weight of the beads.

13. Beads according to claims 1 to 11, **characterized in that** they comprise
   1 - 20 % by weight amorphous cellulose and
   80 - 99 % by weight of a cosmetic ingredient and
   less than 3 % by weight of water based on the weight of the beads.

14. Cosmetic compositions comprising 0.1 to 50 wt % of the beads according to one of claims 1 to 13 based on the weight of the composition.

15. Use of the beads according to claims 1 to 13 as peeling agent or for application of cosmetic ingredients on the skin.

Figure 1:

Figure 2:

Figure 3:

Figure 4:

Fig. 5:

Europäisches
Patentamt

European
Patent Office

Office européen
des brevets

# EUROPEAN SEARCH REPORT

Application Number

EP 19 21 2200

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/074359 A2 (UNILEVER PLC [GB]; UNILEVER NV [NL] ET AL.) 18 June 2009 (2009-06-18) | 1,2,4-8, 11,14 | INV. A61K8/02 A61K8/73 |
| Y | * pages 3,11; claim 1 * | 1-15 | A61Q19/00 |
| Y | CN 107 550 742 A (WUHAN HUALI BIOMATERIAL CO LTD) 9 January 2018 (2018-01-09) * the whole document * | 1-15 | |
| Y | WO 2011/006658 A1 (CLARIANT INT LTD [CH]; LACHMANN ANGELA [DE] ET AL.) 20 January 2011 (2011-01-20) * pages 10-12 * | 1-15 | |
| Y,D | US 2004/131690 A1 (LYNCH MAURICE GERARD [BE]) 8 July 2004 (2004-07-08) * page 3 - page 5 * | 1-15 | |
| Y | JP 2004 331918 A (ASAHI KASEI CHEMICALS CORP) 25 November 2004 (2004-11-25) * the whole document * | 1-15 | TECHNICAL FIELDS SEARCHED (IPC) |
| Y,D | MARTIN GERICKE ET AL: "Functional Cellulose Beads: Preparation, Characterization, and Applications", CHEMICAL REVIEWS, AMERICAN CHEMICAL SOCIETY, US , vol. 113, no. 7 29 March 2013 (2013-03-29), pages 4812-4836, XP002739481, ISSN: 0009-2665, DOI: 10.1021/CR300242J Retrieved from the Internet: URL:http://pubs.acs.org/doi/ipdf/10.1021/cr300242j [retrieved on 2015-05-12] * page 4813 ff * | 1-15 | A61K A61Q |

-/--

The present search report has been drawn up for all claims

| Place of search | Date of completion of the search | Examiner |
|---|---|---|
| Munich | 25 March 2020 | Bader, Karl Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 19 21 2200

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Diana Ciolacu ET AL: "AMORPHOUS CELLULOSE - STRUCTURE AND CHARACTERIZATION", CELLULOSE CHEMISTRY AND TECHNOLOGY Cellulose Chem. Technol, 1 January 2011 (2011-01-01), pages 13-21, XP055679196, Retrieved from the Internet: URL:http://cellulosechemtechnol.ro/pdf/CCT 1-2(2011)/p.13-21.pdf [retrieved on 2020-03-24] * the whole document * | 1-15 | |

-----

TECHNICAL FIELDS
SEARCHED        (IPC)

The present search report has been drawn up for all claims

| Place of search | Date of completion of the search | Examiner |
|---|---|---|
| Munich | 25 March 2020 | Bader, Karl Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 2200

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2020

| Patent document cited in search report | | Publication date | Patent family member(s) | | Publication date |
|---|---|---|---|---|---|
| WO 2009074359 | A2 | 18-06-2009 | AR | 069177 A1 | 06-01-2010 |
| | | | AU | 2008334841 A1 | 18-06-2009 |
| | | | CA | 2707803 A1 | 18-06-2009 |
| | | | CN | 101896157 A | 24-11-2010 |
| | | | EP | 2217199 A2 | 18-08-2010 |
| | | | ES | 2586027 T3 | 11-10-2016 |
| | | | US | 2009155322 A1 | 18-06-2009 |
| | | | WO | 2009074359 A2 | 18-06-2009 |
| | | | ZA | 201003287 B | 31-08-2011 |
| CN 107550742 | A | 09-01-2018 | NONE | | |
| WO 2011006658 | A1 | 20-01-2011 | BR | 112012001088 A2 | 23-02-2016 |
| | | | CN | 102449130 A | 09-05-2012 |
| | | | EP | 2277982 A1 | 26-01-2011 |
| | | | EP | 2454354 A1 | 23-05-2012 |
| | | | EP | 2454355 A1 | 23-05-2012 |
| | | | ES | 2378495 T3 | 13-04-2012 |
| | | | JP | 5669836 B2 | 18-02-2015 |
| | | | JP | 5743229 B2 | 01-07-2015 |
| | | | JP | 2012533528 A | 27-12-2012 |
| | | | JP | 2012533637 A | 27-12-2012 |
| | | | KR | 20120052313 A | 23-05-2012 |
| | | | US | 2012178662 A1 | 12-07-2012 |
| | | | US | 2012183479 A1 | 19-07-2012 |
| | | | WO | 2011006657 A1 | 20-01-2011 |
| | | | WO | 2011006658 A1 | 20-01-2011 |
| US 2004131690 | A1 | 08-07-2004 | NONE | | |
| JP 2004331918 | A | 25-11-2004 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2907498 A1
- US 20180280424 A
- EP 2190882 A
- US 20040131690 A
- WO 2019081246 A

**Non-patent literature cited in the description**

- Functional Cellulose Beads: Preparation, Characterization, and Applications. **GERICKE et al.** Chem. Rev. 013 American Chemical Society Publications, 2013, vol. 113, 4812-4836
- Encapsulation of Either Hydrophilic or Hydrophobic Substances in Spongy Cellulose Particles. **TARO OMURA et al.** ACS Appl. Mater. Interfaces. American Chemical Society, 2017, vol. 9, 944-949
- Continuous Production of Cellulose Microbeads via Membrane Emulsification. **JAMES COOMBS O'BR-IEN et al.** ACS Sustainable Chem. Eng. American Chemical Society, 2017, vol. 5, 5931-5939
- **TARO OMURA et al.** *ACS Appl. Mater. Interfaces,* 2017, vol. 9, 944-949
- **SUNKYU PARK ; JOHN O. BAKER ; MICHAEL E. HIMMEL ; PHILIP A. PARILLA ; DAVID K. JOHN-SON.** Cellulose crystallinity index: Measurement techniques and their impact on interpreting cellulase performance. *Biotechnology for Biofuels,* 2010, vol. 3 (10